# EUROPEAN PATENT SPECIFICATION

(11) **EP 2 918 311 B1**
(45) Date of publication and mention of the grant of the patent: **24.08.2016**
(21) Application number: 15158869.6
(22) Date of filing: 12.03.2015
(51) Int. Cl.: A61N 5/06

(54) **Sun shade for chromotherapy**
Sonnenschirm für Chromotherapie
Parasol pour chromothérapie

(30) Priority: 12.03.2014 IT RM20140124
(43) Date of publication of application: 16.09.2015
(73) Proprietor: Maglia, Venera, 00139 Roma (IT)
(72) Inventor: Maglia, Venera, 00139 Roma (IT)
(74) Representative: Sarpi, Maurizio

(56) References cited:
- US-A- 5 927 311
- US-B1- 6 401 737

## Description

The invention is as defined in the appended claims. The present invention relates to a device for chromo-therapy and more specifically to a particular sun shade to be used in color baths able in filtering the sunlight, irradiating the person, or the concerned body part, by one or more colors so as to influence either the user's mind and physical state. Chromo-therapy is a traditional alternative medicine that uses color as a therapy for the diseases treatment.

In India, Ayurvedic medicine has always considered how colors affect the balance of the chakras, the centers of fine energy associated to the major glands of the body.

The term Cakra, usually transliterated into Chakra, comes from Sanskrit and means "wheel", but it has many exceptions including that of "plexus" or vortex. It's a term used in traditional Indian philosophy and physiology. In the modern Western tradition these chakras are sometimes identified with the name of Centers of Force or Spiritual Senses. An imbalance in chakra level would lead to an energy imbalance in certain associated organs.

Many modern natural therapies, especially Crystal therapy and Reiki, are based on chakras analysis; the reflexology and aromatherapy work on the same meridians, and meditation and visualization based on the colors would give important tools to balance the chakras.

Each chakra has its center in one of the seven endocrine glands of the body endocrine system and their function is to stimulate the gland hormonal production.

It could be said that chakras are the intersection points between the different energy levels of the person, in particular between the physical, mental-emotional and spiritual levels. In each of these levels the vital energy is manifested by a specific body, one of which, the physical one, falls under the sensory perception common to all.

Most theories locates the points out of the seven main chakras along the vertebral spine.

Each chakra vibrates at a certain energy frequency and it is associated with one of the seven primary colors that are: Red, Orange, Yellow, Green, Blue, Indigo, and Violet.

When the chakras are balanced, show all the rainbow colors clearly and uniformly. But when there is some interferences (physical, mental, emotional or spiritual) you can see through the chakras: the energy does not flow regularly along the spine, creating an imbalance. Each of us is subject to a various imbalances kinds that require continuous efforts to be balanced through physical activity or the practice of disciplines that enable the body to regain physical, mental and emotional balance.

Each color has a vibration that affects the molecular structure, the cellular memories of the physical body and also acts on the mental, emotional and spiritual fine bodies.

Several devices to perform color therapy in a closed environment have long been on the market, recurring to the use of lamps of different colors, fed by electric current. However, it should be reminded that we all have at hand the possibility to use a natural source such as the sun, able to provide the full spectrum of colors.

Document US6401737 discloses an umbrella with multiple panels.

Document US5927311 discloses a shelter with exchangeable compartments.

The objective of the invention is therefore to provide a sun shade that can allow to everyone to transform a common sun bath in a color bath, radiating a specific part of their body with a defined color, and/or spreading one or more colors along its vertebral spine so that each chakra of his body is stimulated by its corresponding color.

All without applying to artificial lamps, but only using the transmitted light from the sun.

This has been achieved by providing a sun shade composed by a covering cloth, in a filtering fabric, i.e. able to selectively let passing through the light of at least one primary color. Said cloth is kept stretched between a pair of parallel flexible rods that once curved, are fixed to their ends to two rigid bases dockable to the ground at the ends of any kind of mat, mattress or lightweight cot, preferably roll-up on itself, so as to facilitate the transport.

In a preferred embodiment the two rods are telescopic, the type used for camping tents and made with a lightweight, transparent material, such as fiberglass. Due to their flexibility, curving the two rods give rise to a slight tension towards outside, naturally stretching the cloth from one end to the other.

A peculiar feature of the invention is given by the fact that the cloth is interchangeable, allowing the user to choose between different functional alternatives.

A first embodiment provides the use of a single cloth of mesh or transparent fabric made of natural materials such as cotton, flax, hemp or silk, where the seven primary colors are distributed in a series of seven frames which follow one another from one to the other end of mat or mattress.

A second embodiment provides that the cover is not formed from a single cloth divided into panels containing the seven primary colors but by as many separate panels one for each primary color, which can be fixed independently in the desired position on the supporting structure above mattress or cot where lie those who are subject to treatment. In this case, also non-transparent panels of fabric that will be interposed to the colored transparent panels are provided, so allowing to intercept the sunlight in areas that do not want to radiate, leaving the light to pass only through the desired colors.

Another alternative is given by the use of an entire cloth of transparent material, having a single color.

Yet another variant provides that the cover cloth is made of a synthetic material having special selective possibilities with respect to the wavelengths present in the sunlight, able to let passing through only those of the color someone which want to be subjected.

Further features and advantages of the invention will be apparent from the accompanying drawings which illustrate a preferred embodiment only as a nonlimiting example. In the drawings:
FIG. 1a shows the sun shade as a whole, once assembled;
FIG. 1b shows the sun shade folded for transport;
FIG. 2 shows in perspective the mat or mattress with the dockable bases for the supporting flexible rods and the anchoring pegs to the ground;
FIG. 3 shows the telescopic and flexible pair of rods already curved as an arc;

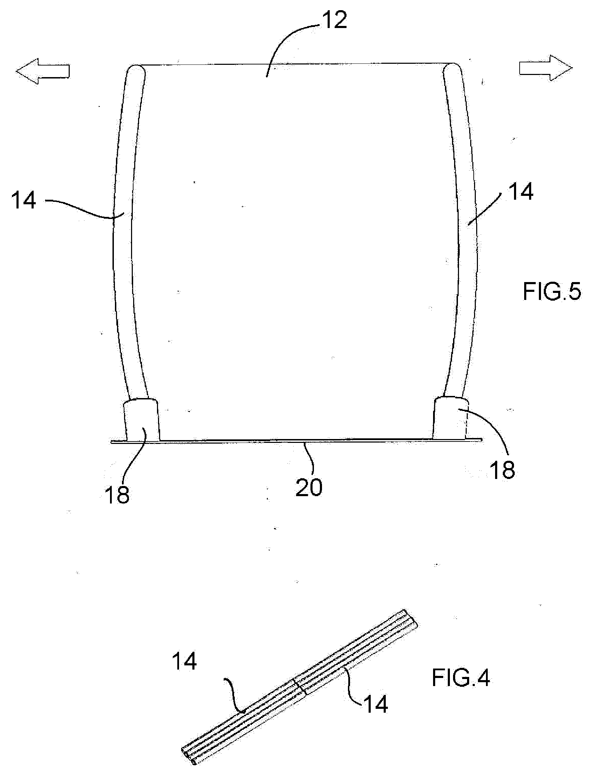

FIG. 4 shows the rods in the state for transport;
FIG. 5 is an elevation view taken from the short side which shows the inclination of the anchoring sites of the flexible rods at the base and the stretching effect for the top cloth once assembled;
FIGS. 6a and 6b show the positioning steps of a covering cloth, once it has been mounted on the flexible rods, containing all the seven primary colors, on the mat or mattress.

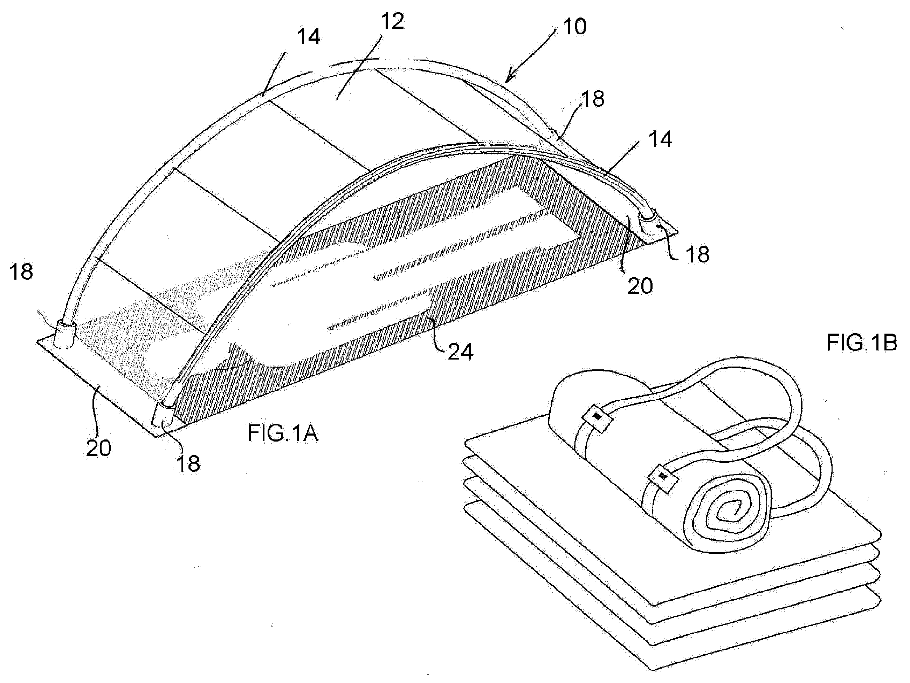

Referring to Fig. 1, the sun shade according to the invention, identified, as a whole, with 10, is constituted by a covering cloth 12 and a supporting structure realized by two flexible rods 14, which are fixed at their ends 16 to the corresponding housing 18 formed in a pair of rigid bases 20 which are positioned across a mat or mattress 24, anchoring them to the ground by means of pegs or the like 22, inserted in holes 23, adopting a characteristic arc shape.

The cloth 12 assembly is obtained by fixing thereof sides to the rods 14 with an usual rapid attachment means or by passing the rods within specific locations or suspension sleeves formed along the edges of the cloth itself.

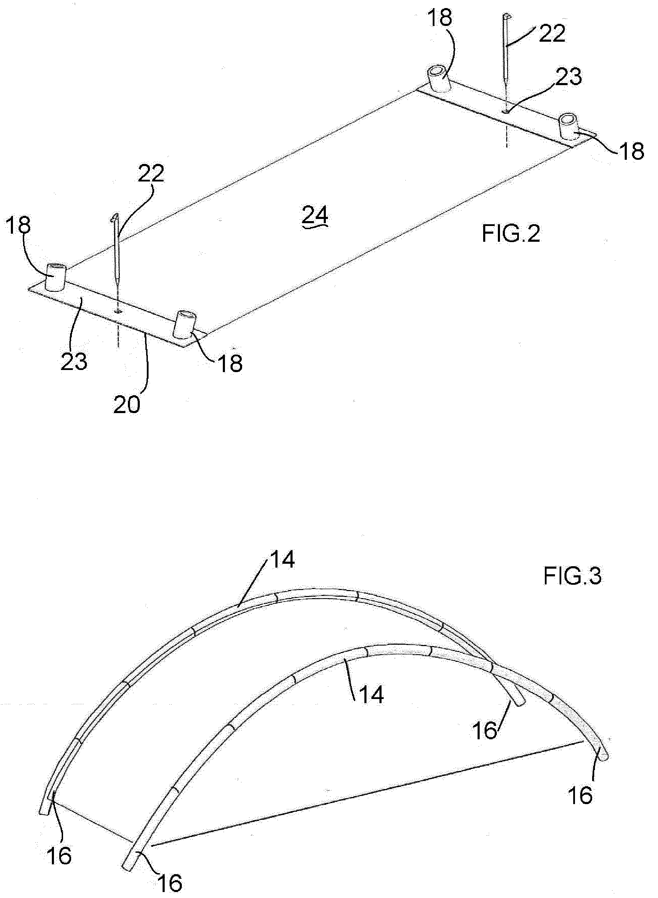

As seen in Fig. 2, for each base 20, the housings 18, in which to insert the ends 16 of the rods 14, are constituted by cylindrical sleeves having the main axis tilted towards outside. Once the cloth 12 has been fixed to the two rods 14, this angle will allow to stretch the cloth itself towards outside, keeping it perfectly tensioned. It will then be the weight of the user's body lying on the mat to prevent the sun shade to move.

In a preferred embodiment, the mattress is represented by a simple mat made by natural materials such as bamboo, which can already be integral, at its short ends, to the bases of the dockable rods.

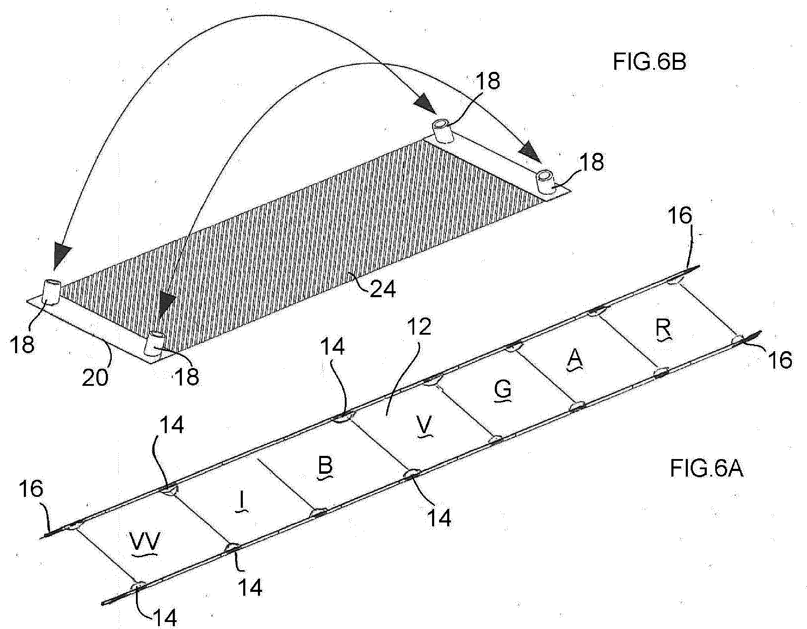

FIG. 6a shows a covering cloth in one piece where there are all the seven primary colors, each of which corresponds to a vibrational frequency, and which are distributed in a succession of boxes indicated with mandala Chakra.

These are:
R- Color Red, corresponding to the first chakra, or center of the base, root or the coccyx which is located at the base of the vertebral spine, where is situated Kundalini;
A- Color Orange, corresponding to the second Chakra, Sacral Center or the Cross, which is located in the lower abdomen, a little down behind the sexual organs about two inches below the navel;
G- Color Yellow, which corresponds to the third chakra, or center of the Solar Plexus or Navel or Wisdom, which is located in the solar plexus;
V- Color Green, which corresponds to the fourth Chakra, or the Heart Center, which is located on the sternum, about the height of the horizontal median line of the breasts;
B- Color Blue, which is the fifth chakra, or center of the Neck, Throat and Communication, which is located at the center of the throat;
I- Color Indigo, which corresponds to the sixth Chakra or Third Eye, the command center, the inner wisdom that is located in the center of the forehead, between the eyebrows, about two fingers above the root of the nose;
VV- Color Purple, which is the seventh Chakra or Crown Center or the Vortex, which is located just above the head, at the top of the skull, and thus does not have a physical location within the body.

As general information, it has been indicated that in chromo-therapy, the use instructions for each color are as follows:
the bath in red color can help in case of fatigue, anemia, weak physical vigor, low blood pressure, insecurity and inability to make decisions;
the orange energy can help in case of: emotional instability, depression, fear, asthma, water retention and loss of appetite;
the yellow bath can help in case of: mental exhaustion, lack of concentration, nervousness, sadness, poor digestion, stomach pain, diabetes and weight problems;
the green energy can help in case of: heart problems, breathing difficulties, asthma, bronchitis, muscle tension, high blood pressure and hypertension;
the blue energy can help in case of: throat problems, thyroid, shoulders, arms, fever, inflammation, wounds, itching, menstrual pain, stress, insomnia and weight problems;
the indigo bath can help in case of: dry skin or wrinkled, abrasions, eczema, hives, psoriasis, headaches, sinusitis, migraine, anxiety and fear, sleep problems;
the purple bath can help in case of: irritability, nervousness, high blood pressure, cramps, sciatica, hair loss, cellulite, varicose veins, bladder problems and infections.

By assembling the cloth containing the seven primary colors of Fig. 6a, with the foresight that the frame R, red, comes to rest on the side of the feet of the person subjected to chromo-therapy, in correspondence of the first Chakra, all remaining colored boxes are going to be placed so as to transmit the own each color vibrations to the corresponding Chakra, with the result to revitalize the entire body, relieving stress, relaxing muscles and refreshing the mind.

Instead, the use of a single color cloth can be determined according to their alright needs, considering the information previously provided. It's clear that the colors of the cloth can be strengthened by darker and/or more intense tonality, and secondary colors like white, brown, pink, turquoise, as well as gold and silver could also be introduced.

The bath lasting of each color, since the sunlight is directly filtered, can vary from one to two hours, and can be repeated also three times a day.

The embodiments aspects and examples which do not fall within the scope of the claims are provided for illustrative purpose only and do not form part of the present invention.

The invention is defined in the claims as follows:

## Claims

1. A removable sun shade for chromo-therapy with seven primary colors, comprising a meshed cloth or fabric configured to let passing through the light of a respective color, and a supporting structure designed to keep said cloth on top of a subject interested by chromo-therapy, wherein said meshed cloth or fabric is divided into a series of seven subsequent colored panels of different color, each panel being colored with one of the seven primary colors;
Said cloth being kept stretched between a pair of parallel flexible rods that are curved to assume a characteristic arc shape, fixing their ends to two rigid bases dockable to ground, covering entirely the subject interested by chromo-therapy laying in a laid position, by which achieving that said colors can be irradiated on whole of the same subject body so allowing each body part to be stimulated by a determinate color.

2. The sun shade for chromo-therapy according to claim 1 **characterized by** the fact that the single cloth divided in panels, with each one of the primary color, is substituted by a plurality of transparent colored panels that are independently fixable on the supporting structure in the desired position on top of the subject body receiving the chromo-therapy.

3. The sun shade for chromo-therapy according to the previous claim **characterized by** the fact that further panels in a covering fabric are provided to be interposed to the colored panels in order to capture the sun light in the areas not desired to be irradiated, letting pass the light only through the desired colors.

4. The sun shade for chromo-therapy according to the preceding claims **characterized by** the fact that the seven primary colors are: Red, Orange, Yellow, Green, Blue, Indigo, and Purple.

## Patentansprüche

1. Abnehmbarer Sonnenschirm zur Chromotherapie mit sieben Primärfarben,
umfassend einen netzartigen Stoff oder Gewebe, der so gestaltet ist, dass Licht einer entsprechenden Farbe durchgelassen wird, und eine Trägerstruktur, die so konstruiert ist, den Stoff über eine an Chromotherapie interessierten Versuchsperson zu halten,
wobei der netzartige Stoff oder Gewebe in eine Reihe von sieben folgenden farbigen Einsatzstreifen verschiedener Farbe geteilt ist, jeder Einsatzstreifen mit einer der sieben Primärfarben gefärbt ist; der Stoff straff gehalten wird zwischen zwei parallelen, flexiblen Stangen, die so gebogen sind, dass sie eine charakteristische Bogenform annehmen, deren Enden an zwei starren, am Boden anlegbaren Grundrahmen fixiert sind, die an Chromotherapie interessierte Person in einer liegenden Position vollständig bedeckt wird, wodurch erreicht wird, dass die Farben auf den ganzen Körper der gleichen Person bestrahlt werden können, womit jeder Körperteil durch eine bestimmte Farbe stimuliert werden kann.

2. Sonnenschirm zur Chromotherapie nach Anspruch 1, **dadurch gekennzeichnet, dass** der in Einsatzstreifen geteilte, einzelne Stoff mit jeweils einer der Primärfarben durch eine Vielzahl transparenter, farbiger Einsatzstreifen ersetzt wird, die unabhängig voneinander an der Trägerstruktur in der gewünschten Position über dem Körper der Person, die die Chromotherapie erhält, befestigbar sind.

3. Sonnenschirm zur Chromotherapie nach dem vorherigen Anspruch, **dadurch gekennzeichnet, dass** weitere Einsatzstreifen in einem Deckgewebe bereitgestellt werden, die zwischen die farbigen Einsatzstreifen gelegt werden, um das Sonnenlicht in den Bereichen einzufangen, in denen Bestrahlung unerwünscht ist, so dass das Licht nur durch die gewünschten Farben durchgelassen wird.

4. Sonnenschirm zur Chromotherapie nach den vorhergehenden Ansprüchen, **dadurch gekennzeichnet, dass** die sieben Primärfarben Rot, Orange, Gelb, Grün, Blau, Indigo und Violett sind.

## Revendications

1. Parasoleil amovible pour chromothérapie avec sept couleurs primaires, comprenant un tissu ou une étoffe à mailles configuré pour laisser passer la lumière d'une couleur respective, et une structure de support conçue pour maintenir ledit tissu sur le dessus d'un sujet concerné par la chromothérapie, dans lequel ledit tissu ou étoffe à mailles est divisé en une série de sept panneaux colorés successifs de différente couleur, chaque panneau étant coloré avec une des sept couleurs primaires ;
ledit tissu étant maintenu étiré entre une paire de tiges flexibles parallèles qui sont courbées pour prendre une forme d'arc caractéristique, en fixant leurs extrémités à deux bases rigides pouvant être ancrées au sol, en couvrant entièrement le sujet concerné par la chromothérapie reposant dans une position couchée, en obtenant ainsi que lesdites couleurs puissent être irradiées sur la totalité du corps de ce sujet en permettant ainsi que chaque partie du corps soit stimulée par une couleur déterminée.

2. Parasoleil pour chromothérapie selon la revendication 1, **caractérisé en ce que** le tissu individuel divisé en panneaux, avec chacune des couleurs primaires, est remplacé par une pluralité de panneaux colorés transparents qui peuvent être fixés indépendamment sur la structure de support dans la position désirée sur le dessus du corps du sujet recevant la chromothérapie.

3. Parasoleil pour chromothérapie selon la revendication précédente, **caractérisé en ce que** d'autres panneaux dans un tissu de couverture sont prévus pour être interposés avec les panneaux colorés de manière à capturer la lumière du soleil dans les zones dans lesquelles l'irradiation n'est pas désirée, en laissant passer la lumière seulement à travers les couleurs désirées.

4. Parasoleil pour chromothérapie selon la revendication précédente, **caractérisé en ce que** les sept coupleurs primaires sont : rouge, orange, jaune, vert, bleu, indigo et violet.
